(19) Europäisches Patentamt
European Patent Office
Office européen des brevets

(11) Veröffentlichungsnummer : **0 108 253**
**B1**

(12) **EUROPÄISCHE PATENTSCHRIFT**

(45) Veröffentlichungstag der Patentschrift : **30.07.86**

(51) Int. Cl.⁴ : **A 61 K 49/02**// C07F9/38

(21) Anmeldenummer : **83109937.9**

(22) Anmeldetag : **05.10.83**

(54) **Technetium-99m-Tetraphosphonate zur szintigraphischen Darstellung RES-haltiger Organe und der Lymphgefässe und Verfahren zu deren Herstellung.**

(30) Priorität : **09.10.82 DE 3237573**

(43) Veröffentlichungstag der Anmeldung : **16.05.84 Patentblatt 84/20**

(45) Bekanntmachung des Hinweises auf die Patenterteilung : **30.07.86 Patentblatt 86/31**

(84) Benannte Vertragsstaaten : **AT BE CH DE FR GB IT LI LU NL SE**

(56) Entgegenhaltungen :
**EP-A- 0 096 930**
**DE-A- 2 618 337**
**FR-A- 2 325 359**
**FR-A- 2 325 360**
**GB-A- 1 448 942**
**US-A- 4 234 562**
**Chemical Abstracts, Band 96, 1982, Seite 264, Nr.48329y,Columbus, Ohio, USA, A.Schwarz et al.:"Relations between chemical structure and skeletal binding of various technetium-99m-labeled phosphonic acids"**
**A. Schwarz et al.: "Nuklearmedizin Suppl. (Stuttg.) 1981, (18) pp. 120-124**
**Die Akte enthält technische Angaben, die nach dem Eingang der Anmeldung eingereicht wurden und die nicht in dieser Patentschrift enthalten sind.**

(73) Patentinhaber : **HOECHST AKTIENGESELLSCHAFT**
**Postfach 80 03 20**
**D-6230 Frankfurt am Main 80 (DE)**

(72) Erfinder : **Kleiner, Hans-Jerg, Dr.**
**Altkönigstrasse 11a**
**D-6242 Kronberg/Taunus (DE)**
Erfinder : **Kloss, Gerhard, Dr.**
**Liederbachstrasse 14**
**D-6233 Kelkheim (Taunus) (DE)**
Erfinder : **Molter, Michael, Dr.**
**Friedrich-Stoltze-Strasse 36**
**D-6231 Schwalbach (DE)**
Erfinder : **Schwarz, Alexander, Dr.**
**Wiesenring 61**
**D-6093 Flörsheim am Main (DE)**
Erfinder : **Thamm, Horst-Dieter, Dr.**
**Parkstrasse 25**
**D-6233 Kelkheim (Taunus) (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Die vorliegende Erfindung bezieht sich auf neue Präparate zur szintigraphischen Darstellung RES-haltiger Organe, insbesondere von Leber und Milz, sowie der Lymphgefäße.

Für die nuklearmedizinische Diagnostik hat sich in den letzten Jahren Technetium-99m wegen seiner günstigen physikalischen Parameter (keine Korpuskularstrahlung, $\gamma$-Energie von 140 keV, Halbwertszeit von 6 Stunden) und der damit verbundenen geringen Strahlenbelastung sowie wegen der einfachen Gewinnung mittels Nuklidgeneratoren weitgehend durchgesetzt.

Das aus solchen Generatoren gewonnene Technetium-99m liegt zunächst als Pertechnetat vor und ist in dieser Form z. B. für die Schilddrüsen- und Hirnszintigraphie geeignet. Zur Diagnostik anderer Organe wurden Präparate entwickelt, die sich mit Technetium-99m gut markieren lassen und die mit hoher Selektivität in bestimmten Organen angereichert werden. So kann man z. B. mit markierten Phosphonsäuren das Skelett oder mit markierten hydrophilen Komplexen wie z. B. Äthylendiamintetraessigsäure die Nieren darstellen.

Für die Markierung von organspezifischen « Transportsubstanzen » mit Technetium-99m muß im allgemeinen das zunächst vorliegende sehr reaktionsträge Pertechnetat ($TcO_4^-$) in eine niedrigere, reaktionsfreudige Oxidationsstufe überführt werden. In dieser reduzierten Form ist Technetium dann reaktionsfreudig und bildet mit den entsprechenden « Transportsubstanzen » vergleichsweise stabile Verbindungen.

Die Reduktion des $TcO_4^-$ kann durch chemische Reduktionsmittel oder elektrolytisch durchgeführt werden ; am häufigsten verwendet man für die Reduktion Zinn(II)-Salze.

Die Reduktion mit Zinn(II) hat den Vorteil, daß man das Reduktionsmittel und die organspezifische Transportsubstanz — im allgemeinen in lyophilisierter Form — in einer Injektionsflasche gemeinsam aufbewahren kann, so daß für die Herstellung des anwendungsfertigen Präparates in der Klinik lediglich eine Lösung mit der gewünschten Tc-99m-Aktivität zugegeben werden muß.

Für die statische Leberszintigraphie verwendet man in aller Regel mit Technetium-99m markierte Kolloide, die nach intravenöser Applikation vom Retikuloendothelial-System (RES) in relativ kurzer Zeit aus dem Blut entfernt werden. Da außer in der Leber auch in der Milz und im roten Knochenmark RES lokalisiert ist, wird selbstverständlich auch in diesen Geweben ein Teil der Radioaktivität angereichert.

Präparate zur Darstellung des RES mit Tc-99m-markierten Kolloiden sind bekannt, z. B.

1. Technetium-99m-Schwefel-Kolloid
2. Technetium-99m-Zinnhydroxid-Kolloid
3. Technetium-99m-Zinn-Phenazon-Kolloid
4. Technetium-99m-Albumin-Millimikrosphären
5. Technetium-99m-Phytat

Alle diese Präparate haben gewisse Nachteile wie zum Beispiel :

Technetium-99m-Schwefel-Kolloid ist bezüglich seiner diagnostischen Eigenschaften ein gut verwendbares Präparat ; zu seiner Herstellung ist aber ein relativ aufwendiges Verfahren notwendig : Die Technetium-99m enthaltende Lösung muß zunächst angesäuert, mit Thiosulfat versetzt, dann gekocht und schließlich neutralisiert werden. Damit ist eine erhöhte Strahlenbelastung des Klinikpersonals verbunden.

Technetium-99m-Zinnhydroxid-Kolloid wird im allgemeinen dadurch hergestellt, daß man ein Zinn(II)-Salz, z. B. $SnCl_2$ oder $SnF_2$, durch Zugabe der das Tc-99m-Pertechnetat enthaltenen Lösung löst, wobei sich beim pH-Wert dieser Lösung — in der Regel zwischen 4, 5 und 7 — Zinnhydroxid bildet. Das durch das Zinn(II) reduzierte Technetium wird dabei mitgefällt. Die Größe der dabei entstehenden Kolloidteilchen hängt von vielen Faktoren wie z. B. Temperatur, Injektionsgeschwindigkeit der Pertechnetatlösung in das Markierungsgefäß und Eluatvolumen ab. Außerdem tritt im Laufe der Zeit eine Aggregation der Teilchen ein, was unter Umständen zu einer nicht beabsichtigten Lungendarstellung führen kann.

Technetium-99m-Zinn-Phenazon-Kolloid wird zwar als präformiertes Kolloid markiert, unter bestimmten Umständen können aber auch hier Teilchenaggregationen auftreten, die gelegentlich unerwünschte Lungendarstellungen verursachen.

Technetium-99m-Albumin-Millimikrosphären bestehen aus denaturierten Albuminteilchen, die eine gewisse Menge Sn(II) zur Reduktion des Pertechnetats enthalten. Die Nachteile dieses Präparates bestehen zum einen darin, daß die Herstellung sehr aufwendig ist, geringfügige Abweichungen von den optimalen Herstellbedingungen führen zu veränderter Teilchengröße und damit unter Umständen zu einer unerwünschten Organverteilung. Zum anderen tritt — wie bei allen Albuminpartikelpräparaten — in Abhängigkeit vom Material der Behälter und Injektionsspritzen eine mehr oder weniger starke Adhäsion an den Wänden von Spritzen, Kanülen und Behältern ein, so daß ein gewisser Anteil der Radioaktivität zurückgehalten wird.

Technetium-99-m-Phytat hat einen vollkommen anderen Wirkungsmechanismus als die bisher beschriebenen Präparate, denn hier liegt im Gegensatz zu jenen in der applikationsfertigen Lösung noch

kein Kolloid vor. Das Kolloid bildet sich erst nach i. v. Injektion im Blut, wahrscheinlich durch Bildung des sehr schwerlöslichen Ca-Salzes. Der Nachteil dieses Präparates liegt vor allem in der verglichen mit den vorgenannten Präparaten geringeren Aktivitätsanreicherung im RES.

Aufgabe der vorliegenden Erfindung ist die Bereitstellung eines Diagnostikums für die Darstellung RES-haltiger Organe und der Lymphgefäße.

Die Erfindung betrifft ein Verfahren zur Herstellung eines Diagnostikums zur Darstellung RES-haltiger Organe, insbesondere der Leber, und der Lymphgefäße, das dadurch gekennzeichnet ist, daß man eine oder mehrere Verbindungen aus der Gruppe Ethan-1,1,2,2-tetraphosphonsäure oder Propan-1,1,3,3-tetraphosphonsäure oder Butan-1,1,4,4-tetraphosphonsäure oder geeignete Salze davon in wäßriger Lösung mit einem Zinn(II)-Salz mischt, die Lösung auf einen pH-Wert zwischen 2 und 10 einstellt, in kleine Portionen vereinzelt, gegebenenfalls die Mischung lyophilisiert und anschließend je nach Verwendungszweck 3,7-3 700 MBq (0,1-100 mCi) Technetium-99m-Pertechnetat in physiologischer Natriumchloridlösung zugibt.

Für die Knochenszintigraphie können Phosphorsäure-Derivate, vor allem organische Phosphonsäure-Derivate eingesetzt werden. Bei der Suche nach geeigneten Derivaten für ein Technetium-99m-Knochendiagnostikum wurde eine Vielzahl von Verbindungen untersucht. In der Arbeit von A. Schwarz und G. Kloss : « Beziehungen zwischen chemischer Struktur und Skelettfixierung verschiedener Tc-99m-Phosphonsäuren » (Nuklearmedizin Suppl. Stuttgart, 1981 (18), S. 120-124) wurde festgestellt, daß 2,4,4-Triphosphonobuttersäure nur vergleichsweise wenig im Knochen, aber relativ stark in der Leber gespeichert wird.

Überraschenderweise wurde nun gefunden, daß sich Ethan-1,1,2,2-tetraphosphonsäure (ETP), Propan-1,1,3,3-tetraphosphonsäure (PTP) und Butan-1,1,4,4-tetraphosphonsäure (BTP) praktisch gar nicht im Knochen, sondern weit überwiegend in der Leber anreichern.

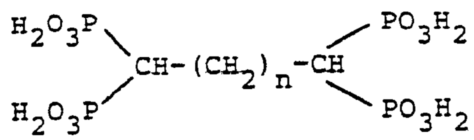

(n = 0 : ETP)
(n = 1 : PTP)
(n = 2 : BTP)

Dieses Ergebnis war in Kenntnis der EP-A-96 930 und der US-A-4 234 562 nicht zu erwarten, wo zumindest Tc-99m-PTP als ein vorteilhaftes Diagnostikum für die Skelett-Szintigraphie beschrieben wird und über die Verteilung dieser Substanz in anderen Organen nichts angegeben wird.

Der Anreicherungsmechanismus dieser Präparate in der Leber ist höchstwahrscheinlich ähnlich wie beim Phytat, jedoch sind die Anreicherungen im RES beim Phytat geringer und die Knochenanreicherungen bei jenem höher.

Eine für die Anwendung beim Menschen geeignete Abfüllung (Markierungseinheit) enthält sinnvollerweise 0,1-200 mg, vorzugsweise 2-50 mg der entsprechenden Tetraphosphonsäure. Die Konzentration an Zinn(II) sollte, bezogen auf die jeweilige Tetraphosphonsäure, bei einem molaren Verhältnis von 1 : 10 bis 1 : 500, vorzugsweise zwischen 1 : 50 und 1 : 150, liegen.

Als Zinn(II)-Salz kann $SnF_2$, $SnSO_4$, SnO, Sn-Acetat, Sn-Oxalat, Sn-tartrat oder ein anderes Salz verwendet werden ; bevorzugt ist das Chlorid, insbesondere $SnCl_2 \times 2H_2O$.

Für die Herstellung der Markierungseinheit ist es von Vorteil, wenn man die entsprechende Tetraphosphonsäure oder ein geeignetes Salz davon in Wasser löst, den pH-Wert der Lösung mit Natronlauge oder Salzsäure auf pH 5-7 einstellt, eine schwach salzsaure Lösung des entsprechenden Zinn(II)-Salzes zugibt, die Lösung mit Wasser auf das gewünschte Volumen bringt, den pH wiederum auf 5-7 einstellt, anschließend die Lösung in kleine Portionen vereinzelt, gegebenenfalls lyophilisiert und schließlich mit Schutzgas wie z. B. Stickstoff überlagert.

Gegenstand der Erfindung ist ferner ein Diagnostikum zur Darstellung RES-haltiger Organe und der Lymphgefäße, das dadurch gekennzeichnet ist, daß es eine oder mehrere Verbindungen aus der Gruppe Ethan-1,1,2,2-tetraphosphonsäure, Propan-1,1,3,3-tetraphosphonsäure oder Butan-1,1,4,4-tetraphosphonsäure, gegebenenfalls ein Zinn(II)-Salz, und Technetium-99m in physiologischer Natriumchloridlösung enthält.

Im Regelfall erhält ein Patient nach der Markierung mit Technetium-99m ein Zehntel bis eine ganze solche « kleine Portion » — Markierungseinheit genannt — intravenös injiziert.

Die Vorteile des hier beschriebenen Diagnostikums auf der Basis der oben genannten Tetraphosphonate gegenüber dem bereits bekannten Phytat gehen aus folgenden Tierversuchen hervor :

Geht man davon aus, daß dem Menschen 2-20 mg ETP, PTP oder Phytat verabreicht werden, so entspricht das bei einem angenommenen Gewicht des Menschen von 80 kg einer Applikationsmenge von 25-250 µg/kg Körpergewicht. Rechnet man auf das Körpergewicht von 200 g schweren Ratten um, so gibt die Applikation von 5-50 µg ETP bzw. PTP bzw. Phytat pro Ratte die Verhältnisse richtig wieder. Vergleichende Untersuchungen mit solchen Mengen haben zu den Organverteilungsdaten in der Tabelle geführt.

Aus den dort dokumentierten Werten lassen sich folgende Schlußfolgerungen ziehen :

— Bei den Substanzen nimmt die Leberspeicherung mit sinkender Substanzmenge ab, während die Aktivitätsanreicherung in Nieren, Femur, Blut und Restkörper parallel dazu ansteigt.

— Bei allen untersuchten Substanzmengen ist die Leber- und Milzanreicherung bei ETP und PTP deutlich höher als bei Phytat.

— Ebenso ist bei allen untersuchten Substanzmengen die Aktivitätsanreicherug in Nieren, Blut, Femur und Restkörper bei ETP und PTP eindeutig geringer als beim Phytat.

Der Vergleich von Tc-99m-ETP, Tc-99m-PTP und Tc-99m-Phytat nach intravenöser Injektion beim Hund zeigt :

— Die Geschwindigkeit der Blutelimination der drei Präparate ist vergleichbar.

Tabelle

Organverteilung von Tc-99m-ETP, Tc-99m-PTP und Tc-99m-Phytat bei Ratten (n = 3) 30 Minuten nach Injektion in Abhängigkeit von der applizierten Substanzmenge (alle Angaben in % der applizierten Aktivitätsmenge)

| Substanzmenge | 50 µg | | | 10 µg | | | 5 µg | | |
|---|---|---|---|---|---|---|---|---|---|
| Verbindung | ETP | PTP | Phytat | ETP | PTP | Phytat | ETP | PTP | Phytat |
| Leber | 96,2 | 91,8 | 83,3 | 95,2 | 84,2 | 68,4 | 93,6 | 82,7 | 62,5 |
| Lunge | 0,18 | 0,30 | 0,23 | 0,21 | 0,45 | 0,32 | 0,32 | 0,43 | 0,40 |
| Milz | 2,10 | 1,31 | 0,60 | 2,13 | 1,23 | 0,38 | 1,30 | 1,10 | 0,25 |
| Nieren | 0,12 | 0,16 | 1,74 | 0,16 | 0,37 | 2,81 | 0,26 | 0,38 | 2,97 |
| Femur/g | + | 0,05 | + | + | 0,088 | 0,74 | + | 0,089 | 1,09 |
| Schilddrüse | 0,03 | 0,05 | 0,04 | 0,03 | 0,04 | 0,03 | 0,04 | 0,05 | 0,03 |
| Blut/ml | 0,02 | 0,05 | 0,19 | 0,04 | 0,11 | 0,33 | 0,04 | 0,086 | 0,62 |
| Restkörper | 7,8 | 9,3 | 22,1 | 11,2 | 16,0 | 29,7 | 10,4 | 18,4 | 36,1 |

+ nicht bestimmt

— Der Aktivitätsabfall über der Leber nach vollzogener Leberanreicherung ist bei ETP und PTP langsamer als beim Phytat. Dies ist vor allem in der klinischen Routine unter Umständen von Vorteil.

— Die Aktivitätsausscheidung mit dem Urin ist bei PTP deutlich geringer als bei Phytat ; 30 min nach Injektion sind bei Phytat bereits 6,6 % der Testdosis, bei PTP nur 0,05 % der Testdosis ausgeschieden.

Aus diesen Tierversuchen läßt sich klar ableiten, das Tc-99m-ETP und Tc-99m-PTP dem Tc-99m-Phytat als Diagnostikum für die Darstellung RES-haltiger Organe eindeutig überlegen sind.

Wie bereits erwähnt, ist der Anreicherungsmechanismus von Tc-99m-EPT und Tc-99m-PTP wahrscheinlich dem des Tc-99m-Phytats vergleichbar. Phytat bildet höchstwahrscheinlich in der Blutbahn mit dem dort vorhandenen Kalzium Kolloide. Da die Teilchenbildung im Blut stark von Kalziumstatus des Patienten, von der Injektionsgeschwindigkeit und einigen weiteren schwer definierbaren Parametern abhängt, kann es sinnvoll sein, das Kolloid bereits in vitro z. B. durch Zugabe von Erdalkaliionen wie Kalzium- oder Bariumionen zu erzeugen. Dieses Kolloid kann dann unter genau definierten Bedingungen hergestellt und markiert werden ; auf diese Weise kann der Einfluß des individuellen Kalziumstatus des Patienten ausgeschaltet werden.

Die Verwendung kleiner Kolloide für die Lymphszintigraphie ist bekannt. Hierfür werden z. Z. vor allem Gold-Kolloid (Au-198) und Tc-99m-$Sb_2S_7$-Kolloid verwendet. Gold-Kolloid ist wegen seiner relativ schlechten strahlenphysikalischen Eigenschaften weniger gut geeignet und Tc-99m-$Sb_2S_7$-Kolloid wird ähnlich wie Tc-99m-Schwefel-Kolloid nach einem vergleichsweise aufwendigen Verfahren hergestellt.

Um die Brauchbarkeit des neuen Diagnostikums für die Lymphszintigraphie zu überprüfen, wurde bei Hunden eine Lymphszintigraphie mit Tc-99m-PTP durchgeführt. Aus den Versuchsergebnissen läßt sich ableiten, daß Tc-99m-PTP auch für die Lymphszintigraphie geeignet ist.

Beispiel 1

1,40 g Ethan-1,1,2,2-tetraphosphonsäure werden in ca. 20 ml bidest. Wasser gelöst und der pH-Wert

der Lösung mit Hilfe von 1 N Natronlauge auf pH 7,0 eingestellt. Das Gesamtvolumen dieser Lösung wird mit bidest. Wasser auf 100 ml gebracht und unter Sauerstoffausschluß fügt man 11 g $SnCl_2 \times 2H_2O$ in 5 ml wäßriger Lösung hinzu. Die so erhaltene klare Lösung wird sterilfiltriert und in Portionen von je 0,2 ml in mit flüssigem Stickstoff vorgekühlte Rollrandfläschen abgefüllt sowie anschließend lyophilisiert. Die Abfüllungen werden mit Stickstoff überlagert und verschlossen. Eine Abfüllung enthält dann ca. 3,3 mg ETP-Na-Salz (entsprechend ca. 2,7 mg ETP) und ca. 11 μg Zinn-II.

Durch Zugabe von 5 ml physiologischer Natriumchloridlösung die 55 MBq (1,5 mCi) Technetium-99m-Pertechnetat enthält, wird die Substanz mit Tc-99m markiert. Nach 15 minütiger Reaktionszeit wurden je 0,1 ml dieser Lösung 3 Sprague-Dawley-Ratten intravenös injiziert. 30 Minuten später wurden die Tiere getötet und die Organverteilung des Tc-99m-ETP gemessen. Folgende Werte (in % der applizierten Dosis) wurden dabei gefunden :

| Leber | Lunge | Milz | Nieren | Schild-drüse | Blut/ml | Rest-körper |
|---|---|---|---|---|---|---|
| 96,2 | 0,18 | 2,1 | 0,12 | 0,03 | 0,02 | 7,8 |

Die Aktivitätsbilanz von 104,5 % wird insbesondere durch Fehler bei der Messung der Restkörper durch die nicht extrakt definierte Geometrie bedingt.

## Beispiel 2

3,5 g 1,1,3,3-Propantetraphosphonsäure (PTP) werden in 250 ml bidest. Wasser gelöst und die Lösung mit 1 N NaOH auf pH 6 eingestellt. Zu dieser Lösung werden 210 mg $SnCl_2 \times 2H_2O$, die in 4,2 ml 0,2 n HCl gelöst sind, zugegeben und der pH wieder auf 6 gestellt. Der Ansatz wird auf 350 ml aufgefüllt und in 1 ml-Portionen vereinzelt. (Alle Operationen bis zum Vereinzeln werden unter $N_2$-Schutzgas durchgeführt). Die Abfüllungen werden eingefroren und gefriergetrocknet. Eine Einheit enthält 10 mg PTP und 0,6 mg $SnCl_2 \times 2H_2O$.

Nach einigen Wochen Lagerzeit wird das Lyophilisat in 10 ml physiologischer Natriumchloridlösung, die 74 MBq (2 mCi) Tc-99m-Pertechnetat enthält, aufgelöst. Nach 30 min werden je 0,1 ml dieser Lösung ca. 200 g schweren Sprague-Dawley-Ratten (n = 6) in die Oberschenkelvene injiziert. Die Tiere werden 30 min p. i. mit Äther getötet und die Aktivitätsverteilung in den Organen bestimmt. Es ergeben sich folgende Durchschnittswerte (in % der applizierten Dosis) :

| Leber | Lunge | Milz | Nieren | Blase mit Urin | Schild-drüse | Blut (1 ml) | Knochen | Rest-körper |
|---|---|---|---|---|---|---|---|---|
| 94,0 | 0,23 | 1,9 | 0,067 | 0,023 | 0,034 | 0,083 | 0,7 | 4,8 |

## Beispiel 3

Eine der gemäß Beispiel 2 hergestellten Markierungseinheiten wird mit 5 ml physiologischer Natriumchloridlösung, die 370 MBq (10 mCi) Tc-99m-Pertechnetat enthält, injektionsfertig gemacht. 1 ml dieser Lösung wird einem 18 kg schweren Hund (Beagle) in die Ohrvene injiziert und der Aktivitätsverlauf über den Bauchraum mit Hilfe einer Gamma-Kamera nach der ROI-Technik verfolgt. Es zeigt sich, daß die Aktivitätsaufnahme in die Leber nach ca. 10 min abgeschlossen ist. Lunge und Nieren werden nicht abgebildet.

Am gleichen Tier werden auch die Blutelimination und die renale Ausscheidung bestimmt. Die Blutclearance verläuft dreiphasig mit den Halbwertszeiten $t_1$ = 3,25 min (97 %), $t_2$ = 20,3 min (2,4 %) und $t_3$ = 500 min (0,6 %). In den ersten 8 Stunden werden etwa 2,3 % der applizierten Aktivität mit dem Urin ausgeschieden.

**Patentansprüche** (für die Vertragsstaaten : BE, CH, DE, FR, GB, IT, LI, LU, NL, SE)

1. Verfahren zur Herstellung eines Diagnostikums zur Darstellung von RES-haltigen Organen oder von Lymphgefäßen mit Tc-99m-Tetraphosphonaten, dadurch gekennzeichnet, daß man eine oder mehrere Verbindungen aus der Gruppe Ethan-1,1,2,2-tetraphosphonsäure (ETP), Propan-1,1,3,3-tetraphosphonsäure (PT) und Butan-1,1,4,4-tetraphosphonsäure (BTP) oder ein geeignetes Salz davon in wäßriger Lösung mit einem Zinn(II)-Salz mischt, die Lösung auf einen pH-Wert zwischen 2 und 10 einstellt, in kleine Portionen vereinzelt und anschließend je nach Verwendungszweck 3,7-3 700 MBq (0,1-100 mCi) Technetium-99m-Pertechnetat in physiologischer Natriumchloridlösung zugibt.

2. Verfahren nach Anspruch 1, dadurch gekennzeichnet, daß die Konzentration der Tetraphosphonate zwischen 0,1 und 200 mg/ml, vorzugsweise zwischen 2 und 50 mg/ml, liegt.

3. Verfahren nach Anspruch 1, dadurch gekennzeichnet, daß das entsprechende Phosphonat und Sn(II) in einem Molverhältnis von 10 : 1 bis 500 : 1, vorzugsweise von 50 : 1 bis 150 : 1, verwendet werden.

4. Verfahren nach den Ansprüchen 1-3, dadurch gekennzeichnet, daß eine Portion (Abfüllung) 0,1 bis 200 mg, vorzugsweise 2-50 mg, des entsprechenden Phosphonats enthält.

5. Verfahren nach den Ansprüchen 1-4, dadurch gekennzeichnet, daß ein pH-Wert zwischen 5 und 7 eingestellt wird.

6. Verfahren nach den Ansprüchen 1-5, dadurch gekennzeichnet, daß man die Abfüllungen vor der Umsetzung mit Technetium-99m-Pertechnetat lyophilisiert.

7. Verfahren nach den Ansprüchen 1-6, dadurch gekennzeichnet, daß als Zinn(II)-Salz das Chlorid, Fluorid, Sulfat, Oxid, Acetat, Oxalat, Tartrat oder ein anderes geeignetes Salz verwendet wird.

8. Diagnostikum zur Darstellung RES-haltiger Organe und der Lymphgefäße, das dadurch gekennzeichnet ist, daß es eine oder mehrere mit Technetium-99m markierte Verbindung aus der Gruppe Ethan-1,1,2,2-tetraphosphonsäure (ETP) und Butan-1,1,4,4-tetraphosphonsäure (BTP) oder ein geeignetes Salz davon in physiologischer Natriumchloridlösung enthält.

9. Diagnostikum gemäß Anspruch 8 zur Darstellung der Leber.

10. Verfahren nach den Ansprüchen 1-7, dadurch gekennzeichnet, daß man der Lösung zusätzlich Erdalkaliionen zusetzt.

11. Verfahren nach Anspruch 10, dadurch gekennzeichnet, daß als Erdalkaliion Magnesium, Barium oder vorzugsweise Kalzium verwendet wird.

12. Verfahren nach den Ansprüchen 10 und 11, dadurch gekennzeichnet, daß man das Erdalkaliion in molaren Mengenverhältnissen, bezogen auf die entsprechende Tetraphosphonsäure, zugibt.

13. Diagnostikum zur Darstellung RES-haltiger Organe und der Lymphgefäße, dadurch gekennzeichnet, daß es eine oder mehrere mit Technetium-99m markierte Verbindungen aus der Gruppe Ethan-1,1,2,2-tetraphosphonsäure (ETP), Propan-1,1,3,3-tetraphosphonsäure (PTP) und Butan-1,1,4,4-tetraphosphonsäure (BTP) oder ein geeignetes Salz davon und zusätzliche Erdalkaliionen, insbesondere Ca-Ionen, in physiologischer Natriumchloridlösung enthält.

**Patentansprüche** (für den Vertragsstaat AT)

1. Verfahren zur Herstellung eines Diagnostikums zur Darstellung von RES-haltigen Organen oder von Lymphgefäßen mit Tc-99m-Tetraphosphonaten, dadurch gekennzeichnet, daß man eine oder mehrere Verbindungen aus der Gruppe Ethan-1,1,2,2-tetraphosphonsäure (ETP), Propan-1,1,3,3-tetraphosphonsäure (PTP) und Butan-1,1,4,4-tetraphosphonsäure (BTP) oder ein geeignetes Salz davon in wäßriger Lösung mit einem Zinn(II)-Salz mischt, die Lösung auf einen pH-Wert zwischen 2 und 10 einstellt, in kleine Portionen vereinzelt und anschließend je nach Verwendungszweck 3,7-3 700 MBq (0,1-100 mCi) Technetium-99m-Pertechnetat in physiologischer Natriumchloridlösung zugibt.

2. Verfahren nach Anspruch 1, dadurch gekennzeichnet, daß die Konzentration der Tetraphosphonate zwischen 0,1 und 200 mg/ml, vorzugsweise zwischen 2 und 50 mg/ml, liegt.

3. Verfahren nach Anspruch 1, dadurch gekennzeichnet, daß das entsprechende Phosphonat und Sn(II) in einem Molverhältnis von 10 : 1 bis 500 : 1, vorzugsweise von 50 : 1 bis 150 : 1, verwendet werden.

4. Verfahren nach den Ansprüchen 1-3, dadurch gekennzeichnet, daß eine Portion (Abfüllung) 0,1 bis 200 mg, vorzugsweise 2-50 mg, des entsprechenden Phosphonats enthält.

5. Verfahren nach den Ansprüchen 1-4, dadurch gekennzeichnet, daß ein pH-Wert zwischen 5 und 7 eingestellt wird.

6. Verfahren nach den Ansprüchen 1-5, dadurch gekennzeichnet, daß man die Abfüllung vor der Umsetzung mit Technetium-99m-Pertechnetat lyophilisiert.

7. Verfahren nach den Ansprüchen 1-6, dadurch gekennzeichnet, daß als Zinn(II)-Salz das Chlorid, Fluorid, Sulfat, Oxid, Acetat, Oxalat, Tartrat oder ein anderes geeignetes Salz verwendet wird.

8. Verfahren nach den Ansprüchen 1-7, dadurch gekennzeichnet, daß man der Lösung zusätzlich Erdalkaliionen zusetzt.

9. Verfahren nach Anspruch 8, dadurch gekennzeichnet, daß als Erdalkaliion Magnesium, Barium oder vorzugsweise Kalzium verwendet wird.

10. Verfahren nach den Ansprüchen 8 und 9, dadurch gekennzeichnet, daß man das Erdalkaliion in molaren Mengenverhältnissen, bezogen auf die entsprechende Tetraphosphonsäure, zugibt.

**Claims** (for the Contracting States : BE, CH, DE, FR, GB, IT, LI, LU, NL, SE)

1. A process for the preparation of a diagnostic agent for the visualization of RES-containing organs or of lymph vessels using $^{99m}Tc$ tetraphosphonates which comprises mixing one or more compounds of the group comprising ethane-1,1,2,2-tetraphosphonic acid (ETP), propane-1,1,3,3-tetraphosphonic acid (PTP) and butane-1,1,4,4-tetraphosphonic acid (BTP) or a suitable salt thereof, in aqueous solution, with a tin(II) salt, adjusting the pH of the solution to a value between 2 and 10, preparing individual small

portions and then adding, depending on the purpose for which it is to be used, 3.7-3,700 MBq (0.1-100 mCi) of technetium-99m-pertechnetate in physiologic saline.

2. The process as claimed in claim 1, wherein the concentration of the tetraphosphonates is between 0.1 and 200 mg/ml, preferably between 2 and 50 mg/ml.

3. The process as claimed in claim 1, wherein the appropriate phosphonate and Sn(II) are used in a molar ration of 10 : 1 to 500 : 1, preferably of 50 : 1 to 150 : 1.

4. The process as claimed in any of claims 1-3, wherein a portion (contents of the container) contains 0.1 to 200 mg, preferably 2-50 mg, of the appropriate phosphonate.

5. The process as claimed in any of claims 1-4, wherein the pH is adjusted to between 5 and 7.

6. The process as claimed in any of claims 1-5, wherein the contents of the containers are freeze-dried before reaction with technetium-99m-pertechnetate.

7. The process as claimed in any of claims 1-6, wherein the tin(II) salt used is the chloride, fluoride, sulfate, oxide, acetate, oxalate, tartrate or another suitable salt.

8. A diagnostic agent for the visualization of RES-containing organs and the lymph vessels, which contains one or more compounds labeled with technetium-99m from the group comprising ethane-1,1,2,2-tetraphosphonic acid (ETP) and butane-1,1,4,4-tetraphosphonic acid (BTP) or a suitable salt thereof in physiologic saline.

9. The diagnostic agent as claimed in claim 8 for visualization of the liver.

10. The process as claimed in any of claims 1-7, wherein alkaline earth ions are also added to the solution.

11. The process as claimed in claim 10, wherein the alkaline earth ion used is magnesium, barium, or, preferably, calcium.

12. The process as claimed in either of claims 10 or 11, wherein the alkaline earth ion is added in equimolar amounts relative to the appropriate tetraphosphonic acid.

13. A diagnostic agent for the visualization of RES-containing organs and the lymph vessels which contains one or more compounds labelled with technetium-99m comprising ethane-1,1,2,2-tetraphosphonic acid (ETP), propane-1,1,3,3-tetraphosphonic acid (PTP) and butane-1,1,4,4-tetraphosphonic acid (BTP) or a suitable salt thereof and additional alkaline earth ions, preferably calcium ions, in physiologic saline.

**Claims** (for the Contracting State AT)

1. A process for the preparation of a diagnostic agent for the visualization of RES-containing organs or of lymph vessels using $^{99m}$Tc tetraphosphonates, which comprises mixing one or more compounds of the group comprising ethane-1,1,2,2-tetraphosphonic acid (ETP), propane-1,1,3,3-tetraphosphonic acid (PTP) and butane-1,1,4,4-tetraphosphonic acid (BTP) or a suitable salt thereof, in aqueous solution, with a tin(II) salt, adjusting the pH of the solution to a value between 2 and 10, preparing individual small portions and then adding, depending on the purpose for which it is to be used, 3.7-3,700 MBq (0.1-100 mCi) of technetium-99m-pertechnetate in physiologic saline.

2. The process as claimed in claim 1, wherein the concentration of the tetraphosphonates is between 0.1 and 200 mg/ml, preferably between 2 and 50 mg/ml.

3. The process as claimed in claim 1, wherein the appropriate phosphonate and Sn(II) are used in a molar ratio of 10 : 1 to 500 : 1, preferably of 50 : 1 to 150 : 1.

4. The process as claimed in any of claims 1-3, wherein a portion (contents of the container) contains 0.1 to 200 mg, preferably 2-50 mg, of the appropriate phosphonate.

5. The process as claimed in any of claims 1-4, wherein the pH is adjusted to between 5 and 7.

6. The process as claimed in any of claims 1-5, wherein the contents of the containers are freeze-dried, before reaction with technetium-99m-pertechnetate.

7. The process as claimed in any of claims 1-6, wherein the tin(II) salt used is the chloride, fluoride, sulfate, oxide, acetate, oxalate, tartrate or another suitable salt.

8. The process as claimed in any of claims 1-7, wherein alkaline earth ions are also added to the solution.

9. The process as claimed in claim 8, wherein the alkaline earth ion used is magnesium, barium, or, preferably, calcium.

10. The process as claimed in either of claims 8 to 9, wherein the alkaline earth ion is added in equimolar amounts relative to the tetraphosphonic acid.

**Revendications** (pour les Etats contractants : BE, CH, DE, FR, GB, IT, LI, LU, NL, SE)

1. Procédé pour la préparation d'un agent de diagnostic pour la visualisation d'organes du système réticulo-endothélial ou de vaisseaux lymphatiques avec des tétraphosphonates marqués avec Tc-99m, caractérisé en ce qu'on mélange un ou plusieurs composés choisis parmi l'acide éthane-1,1,2,2-tétraphosphonique (ETP), l'acide propane-1,1,3,3-tétraphosphonique (ETP), l'acide propane-1,1,3,3-tétraphosphonique (PTP) et l'acide butane-1,1,4,4-tétraphosphonique (BTP) ou un de leurs sels en solution aqueuse, avec un sel d'étain (II), on ajuste la valeur du pH de la solution entre 2 et 10, on divise en petites portions et selon l'application on ajoute de 3,7 à 3 700 MBq (0,1 à 100 mCi) de pertechnétate marqué à Tc-99m dans une solution physiologique de chlorure de sodium.

2. Procédé selon la revendication 1, caractérisé en ce que la concentration des tétraphosphonates est comprise entre 0,1 et 200 mg/ml, de préférence entre 2 et 50 mg/ml.

3. Procédé selon la revendication 1, caractérisé en ce que le phosphonate choisi et Sn (II) sont utilisés dans un rapport molaire de 10 : 1 à 500 : 1, de préférence de 50 : 1 à 150 : 1.

4. Procédé selon l'une quelconque des revendications 1 à 3, caractérisé en ce qu'une portion (unité de remplissage) contient de 0,1 à 200 mg, de préférence de 2 à 50 mg du phosphonate choisi.

5. Procédé selon l'une quelconque des revendications 1 à 4, caractérisé en ce qu'on ajuste la valeur du pH entre 5 et 7.

6. Procédé selon l'une quelconque des revendications 1 à 5, caractérisé en ce qu'on lyophilise les unités de remplissage avant la réaction avec le pertechnétate marqué à Tc-99m.

7. Procédé selon l'une quelconque des revendications 1 à 6, caractérisé en ce que, comme sel d'étain (II), on utilise le chlorure, le fluorure, le sulfate, l'oxyde, l'acétate, l'oxalate, le tartrate ou un autre sel approprié.

8. Agent de diagnostic pour la visualisation d'organes du système réticulo-endothélial et des vaisseaux lymphatiques, caractérisé en ce qu'il contient un ou plusieurs composés marqués avec le technétium-99m, choisis parmi l'acide éthane-1,1,2,2-tétraphosphonique, l'acide propane-1,1,3,3-tétraphosphonique et l'acide butane-1,1,4,4-tétraphosphonique ou un de leurs sels appropriés dans une solution physiologique de chlorure de sodium.

9. Agent de diagnostic selon la revendication 8 pour la visualisation du foie.

10. Procédé selon l'une quelconque des revendications 1 à 7, caractérisé en ce qu'à la solution on ajoute en outre des ions alcalinoterreux.

11. Procédé selon la revendication 10, caractérisé en ce que comme ion alcalinoterreux, on utilise le magnésium, le baryum ou de préférence le calcium.

12. Procédé selon l'une des revendications 10 et 11, caractérisé en ce qu'on ajoute l'ion alcalinoterreux en des rapports molaires quantitatifs, par rapport à l'acide tétraphosphonique choisi.

13. Agent de diagnostic pour la visualisation d'organes du système réticulo-endothélial et des vaisseaux lymphatiques, caractérisé en ce qu'il contient un ou plusieurs composés marqués au technétium-99m, choisis parmi l'acide éthane-1,1,2,2-tétraphosphonique (ETP), l'acide propane-1,1,3,3-tétraphosphonique (PTP) et l'acide butane-1,1,4,4-tétraphosphonique (BTP) ou un de leurs sels appropriés, et en outre des ions alcalinoterreux, en particulier des ions Ca, dans une solution physiologique de chlorure de sodium.

**Revendications** (pour l'Etat contractant AT)

1. Procédé pour la préparation d'un agent de diagnostic pour la visualisation d'organes du système réticulo-endothélial ou des vaisseaux lymphatiques avec des tétraphosphonates marqués à Tc-99m, caractérisé en ce qu'on mélange un ou plusieurs composés choisis parmi l'acide éthane-1,1,2,2-tétraphosphonique (ETP), l'acide propane-1,1,3,3-tétraphosphonique (PTP) et l'acide butane-1,1,4,4-tétraphosphonique (BTP) ou un de leurs sels appropriés en solution aqueuse, avec un sel d'étain (II), on ajuste le pH de la solution à une valeur comprise entre 2 et 10, on divise en petites portions et finalement selon l'application on ajoute de 3,7 à 3 700 MBq (0,1-100 mCi) de pertechnétate marqué au technétium-99m, dans une solution physiologique de chlorure de sodium.

2. Procédé selon la revendication 1, caractérisé en ce que la concentration des tétraphosphonates est comprise entre 0,1 et 200 mg/ml, de préférence entre 2 et 50 mg/ml.

3. Procédé selon la revendication 1, caractérisé en ce que le phosphonate choisi et Sn(II) sont utilisés dans un rapport molaire de 10 : 1 à 500 : 1, de préférence de 50 : 1 à 150 : 1.

4. Procédé selon l'une quelconque des revendications 1 à 3, caractérisé en ce qu'une portion (unité de remplissage) contient de 0,1 à 200 mg, de préférence de 2 à 50 mg du phosphonate choisi.

5. Procédé selon l'une quelconque des revendications 1 à 4, caractérisé en ce qu'on ajuste le pH à une valeur comprise entre 5 et 7.

6. Procédé selon l'une quelconque des revendications 1 à 5, caractérisé en ce qu'on lyophilise l'unité de remplissage avant la réaction avec le pertechnétate marqué au technétium-99m.

7. Procédé selon l'une quelconque des revendications 1 à 6, caractérisé en ce que, comme sel d'étain (II), on utilise le chlorure, le fluorure, le sulfate, l'oxyde, l'acétate, l'oxalate, le tartrate ou un autre sel approprié.

8. Procédé selon l'une quelconque des revendications 1 à 7, caractérisé en ce qu'à la solution on ajoute encore des ions alcalinoterreux.

9. Procédé selon la revendication 8, caractérisé en ce que, comme ions alcalinoterreux, on utilise le magnésium, le baryum ou de préférence le calcium.

10. Procédé selon l'une des revendications 8 et 9, caractérisé en ce qu'on ajoute l'ion alcalinoterreux en des rapports quantitatifs molaires, par rapport à l'acide tétraphosphonique choisi.